# EUROPÄISCHE PATENTANMELDUNG

(11) **EP 1 772 165 A1**
(43) Veröffentlichungstag der Anmeldung: **11.04.2007**
(21) Anmeldenummer: 05021988.0
(22) Anmeldetag: 08.10.2005
(51) Int. Cl.: A61M 11/00

(54) **Inhalationsgerät**

(71) Anmelder: Cegla, Ulrich, 56140 Montabaur (DE)
(72) Erfinder: Cegla, Ulrich, 56140 Montabaur (DE)
(74) Vertreter: Engelhardt, Volker

(57) **Zusammenfassung**

Ein Inhalationsgerät (1) zum Einatmen von Luft-Nebel- oder Gas-Gemischen (27), insbesondere von medizinischen Aerosolen, bestehend aus einem Aerosolspender (26,27,28) und einem an diesen angeschlossenen Verbindungselement (21) durch dessen Kanal (35) das Aerosol dem Nasen- Rachen-Raum (3 bzw. 4) eines Menschen (2) zuführbar ist. An das Verbindungselement ist ein Atmungs-Therapiegerät (11) angeschlossen, welches beim Ausatmen ein oszillierender Luftwiderstand erzeugt der im Nasen-Rachen-Raum einen oszillierenden Überdruck bildet. In dem Kanal (35) des Verbindungselementes, zwischen dem Aerosolspender und dem Anschluss für das Atmungs-Therapiegerät, ist ein Rückschlagventil (32) angeordnet, das während des Einatmens geöffnet und das während des Ausatmens geschlossen ist. Alternativ weist das Verbindungselement zwei unabhängig voneinander verlaufende Kanäle (33,36) auf, wobei der eine Kanal zwischen dem Aerosolspender und dem Nasen- Rachen-Raum angeordnet ist, in dem ein vorbestimmbarer Überdruck herrscht, und der andere Kanal von dem Nasen-Rachen-Raum in das Therapiegerät einmündet.

## Beschreibung

Die Erfindung bezieht sich auf ein Inhalationsgerät zum Einatmen von Luft-Nebel- oder Gasgemischen, insbesondere von medizinischen Aerosolen, in den Nasen-Rachen-Raum eines Menschen, bestehend aus einem das Aerosol aufnehmenden als Vorratsbehälter ausgebildeten Aerosolspender und einem an diesen angeschlossenen Verbindungselement, durch dessen Kanal das Aerosol dem Nasen-Rachen-Raum zuführbar ist sowie aus einem Verfahren zur Anwendung des Inhalationsgerätes gemäß des Oberbegriffs von Patentanspruch 17.

Solche Inhalationsgeräte werden auch als Nasenduschen bezeichnet, durch die sogenannte Aerosole als Gemisch in den Nasenraum eingesprüht werden. Das in den Nasenraum eingespritzte Aerosol bedeckt die Nasenwand und soll in die Nasennebenhöhlen gelangen, um die Schleimhäute zu massieren. Auch ist der Einsatz solcher Aerosole für die Behandlung von Erkältungen der Nasennebenhöhlen und des Rachenraums wie z.B. chronische Rhinitis, chronische Sinusitis sowie des Postnasaldrip-Syndroms bekannt. Über die Nase können dem menschlichen Körper auch therapeutische Aerosole zugeführt werden, die über die Nasenschleimhäute resorbiert werden und in den menschlichen Körper gelangen, wie z.B. Insulin.

Als nachteilig bei der Verwendung der bekannten Inhalationsgeräte hat sich herausgestellt, dass beim Ausatmen des Aerosols bzw. des Gemisches dieses ohne Kraftanstrengung durch den Patienten ausgebracht wird, so dass dieses relativ schnell den Nasen- und Mundhöhlenraum verlässt. Dieses Aerosol gelangt demnach nicht in den gesamten Nasenhöhlenraum und insbesondere nicht in die Nasennebenhöhlen, wenn die Zugänge zu diesen verstopft sind, denn durch das Ausblasen ohne Kraftanstrengung werden die Zugänge zu den Nasennebenhöhlen nicht geöffnet.

Aus der EP 0 681 853 A1 ist ein Atmungs-Therapiegerät bekannt geworden, durch dessen Verwendung beim Ausatmen ein oszillierend verlaufender Luftwiderstand erzeugbar ist, durch den in den Nasenraum ein oszillierender Überdruck entsteht.

Wenn das Inhalationsgerät zusammen mit dem Atmungs-Therapiegerät verwendet wird, hat sich als nachteilig herausgestellt, dass der Patient zunächst das Inhalationsgerät in die Nasenöffnung einführen muss und nachdem das Aeorsol in den Nasenraum eingeatmet wurde, ist das Inhalationsgerät aus der Nasenöffnung zu entfernen und statt dessen das Atmungs-Therapiegerät in diese einzuschieben. Zwischen diesen Auswechselvorgängen hat der Patient die Atmung einzustellen, um das Aerosol im Nasenraum zu belassen. Dies erfolgt jedoch oftmals nicht, denn der Patient leidet an verschiedenen Erkrankungen, beispielsweise Bronchitis, und ist daher möglicherweise nicht in der Lage, insbesondere über einen längeren Zeitraum, die Luft anzuhalten.

Auch ist die Handhabung von zwei medizinischen Geräten umständlich und zeitaufwendig.

Der Erfindung liegt daher die Aufgabe zugrunde, ein Inhalationsgerät der eingangs genannten Gattung derart weiterzubilden, dass durch dieses sowohl ein Aerosol oder ein beliebiges Gemisch in den Nasen-Rachen-Raum eingeatmet werden kann und dass beim Ausatmen im Nasen-Rachen-Raum ein oszillierend verlaufender Überdruck gebildet wird, durch den das Aerosol bzw. das Gemisch im gesamten Nasen- Rachen-Raum verteilt wird und auch in schwer zugängliche Gebiete, beispielsweise der Nasennebenhöhlen, gelangt. Die Handhabung des erfindungsgemäßen Inhalationsgerätes soll einfach sein, und durch dessen Benutzung sollen keine zeitlichen Verzögerungen während der Behandlungsphase eintreten.

Diese Aufgaben werden erfindungsgemäß dadurch gelöst, dass an das Verbindungselement ein Atmungs-Therapiegerät angeschlossen ist, dass durch das Atmungs-Therapiegerät beim Ausatmen ein oszillierender Luftwiderstand erzeugbar ist, der im Nasen-Rachen-Raum einen Überdruck bildet und dass in dem Kanal des Verbindungselementes zwischen dem Aerosolspender und dem Anschluss für das Atmungs-Therapiegerät ein Rückschlagventil angeordnet ist, das während des Einatmens geöffnet und das während des Ausatmens geschlossen ist, oder dass das Verbindungselement zwei unabhängig voneinander verlaufende Kanäle aufweist, dass der eine Kanal zwischen dem Aerosolspender und dem Nasen-Rachen-Raum angeordnet ist, in dem ein vorbestimmbarer Überdruck herrscht, und dass der andere Kanal von dem Nasen-Rachen-Raum in das Atmungs-Therapiegerät einmündet.

Die Handhabung des Inhalationsgerätes erfolgt erfindungsgemäß durch die nachfolgend angegebenen Verfahrensschritte:
- Ausblasen des eingeatmeten Aerosols bzw. des Gemisches durch ein Therapiegerät, durch das ein oszillierender Luftwiderstand erzeugt wird,
- Erhöhung des Luftdruckes in dem Nasen- Rachen-Raum durch den von dem Therapiegerät erzeugten Luftwiderstand und
- Verteilen des Aerosols bzw. des Gemisches im Nasen-Rachen-Raum durch die Druckoszillationen während des Ausatmungsvorganges.

Dadurch, dass das Verbindungselement aus einem zweiteiligen Schlauch und einer Weiche besteht, die drei Anschlüsse aufweist, dass an zwei Anschlüssen der Weiche jeweils ein Teil des Schlauches und mit dem dritten Anschluss der Weiche das Atmungs-Therapiegerät verbunden ist, wird gewährleistet, dass der konstruktive Aufbau des Inhalationsgerätes einfach gestaltet ist, um die Montage und Demontage zu Reinigungszwecken zuverlässig bewerkstelligen zu können.

Weitere vorteilhafte Ausgestaltungen des erfindungsgemäßen Inhalationsgerätes ergeben sich aus den Unteransprüchen.

Mittels des erfindungsgemäßen Inhalationsgerätes wird demnach erreicht, dass der Patient lediglich ein medizinisches Gerät verwenden muss, um eine optimale Verteilung bzw. Deposition des Gemisches im Nasen-Rachen-Raum, und zwar auch in den Nasennebenhöhlen, die unter Umständen verschlossen sind, zu erzielen. Die Handhabung erfolgt unmittelbar durch den Patienten, denn dieser kann das Inhalationsgerät manuell vor seinem Gesichtsfeld mit einer Hand halten, das Verbindungselement durch einen entsprechend ausgestalteten Ansatz in eine Nasenöffnung einführen und mit der anderen Hand die andere Nasenöffnung verschließen. Das Gerät ist beim Ein- und Ausatmen nicht zu wechseln, denn das angeschlossene Atmungs-Therapiegerät wird durchströmt, wenn der Patient das eingeatmete Aerosol ausbläst. Der dabei erzeugte oszillierende Luftwiderstand im Atmungs-Therapiegerät führt dazu, dass im Nasen-Rachen-Raum des Patienten ein oszillierender Überdruck entsteht, durch den die Nasennebenhöhleneingänge geöffnet werden, wodurch das Gemisch bzw. Aerosol auch in diese gelangt, dabei kommt es zu einer besseren Deposition des Aerosols.

In der Zeichnung sind zwei erfindungsgemäße Ausführungsbeispiele dargestellt, die nachfolgend näher erläutert werden.

Im Einzelnen zeigt in perspektivischer Ansicht:

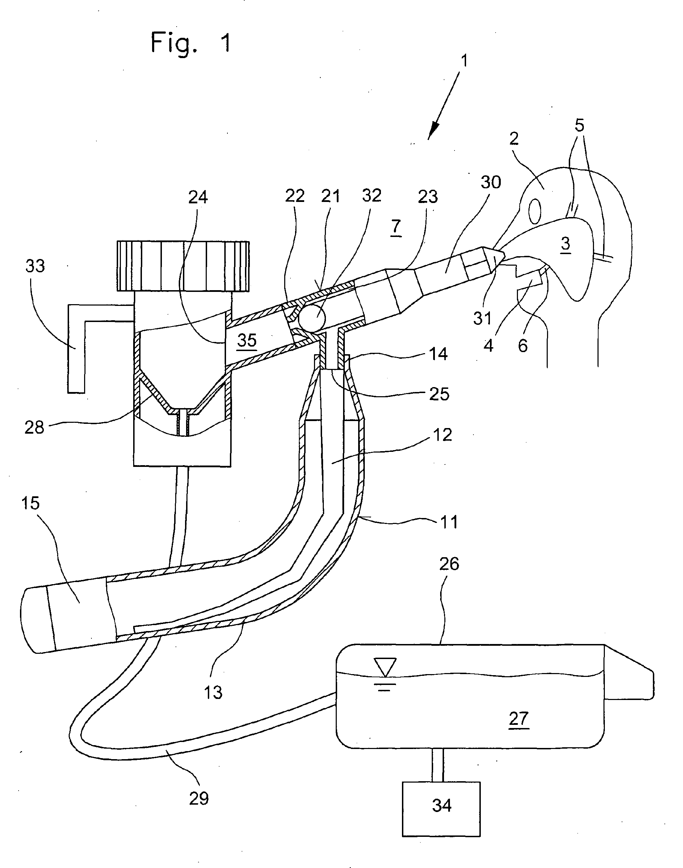

- Figur 1: ein erstes Ausführungsbeispiel eines Inhalationsgerätes, durch das ein in einem Aerosolspender gelagertes Aerosol dem Nasen-Rachen-Raum eines Menschen zugeführt wird und an dem ein Atmungstherapiegerät angeschlossen ist, durch das ein oszillierender Luftwiderstand beim Ausatmen des Aerosols erzeugbar ist, die von einander durch ein Rückschlagventil getrennt sind und

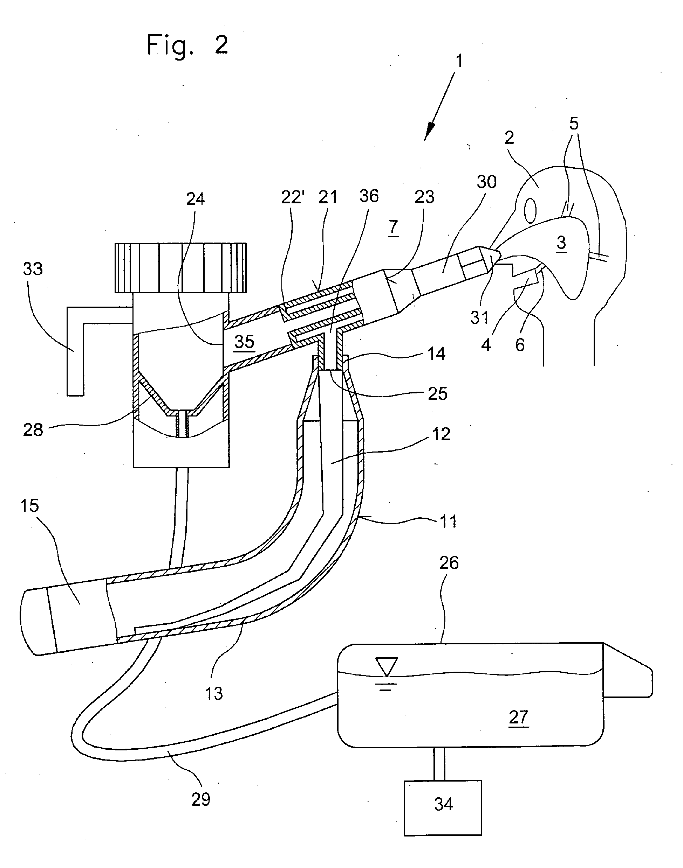

- Figur 2: ein zweites Ausführungsbeispiel eines Inhalationsgerätes, durch das ein in einem Aerosolspender gelagertes Aerosol in einem Nasen-Rachen-Raum eines Menschen zugeführt wird und an dem ein Atmungstherapiegerät angeschlossen ist, durch das ein oszillierender Luftwiderstand beim Ausatmen des Aerosols erzeugbar ist, mit zwei getrennten Kanälen für den Ein- und Ausatmungsvorgang.

In den Figuren 1 und 2 ist ein Inhalationsgerät 1 dargestellt, mittels dem ein Luft-Nebel- oder Gasgemisch 27, insbesondere ein Aerosol, in den Nasen-Rachen-Raum 3 und/oder 4 eines Menschen 2 eingeatmet werden kann. Das Aerosol 27 dient zur Therapie der Nasen-Rachen-Schleimhäute und kann auch zu Therapiezwecken gegen chronische Rhinitis, chronischer Sinusitis oder zum Anfeuchten der Schleimhäute sowie zur Behandlung des sogenannten Postnatal-drip-Syndroms verwendet werden. Das Aerosol 27 kann auch aus Insulin oder anderen Pharmaka bestehen, die zur systemischen Therapie über naso-broncho-pulmunalen Resorption dienen.

Um eine optimale und zielorientierte medizinische Behandlung des Patienten 2 zu gewährleisten, ist es erforderlich, dass das in die Nase oder Nasenrachen 3 bzw. 4 eingeatmete Aerosol 27 ausreichend verteilt wird. Die Verteilung des Aerosols 27, nachdem dieses inhaliert wurde, wird durch ein an das Inhalationsgerät 1 angeschlossenes Atmungstherapiegerät 11 bewerkstelligt, durch das die eingeatmete Luft sowie das Aeorosol 27 vom Menschen 2 ausgeblasen wird.

In Figur 1 ist gezeigt, dass das Inhalationsgerät 1 aus einem Verbindungselement 21 besteht, das an einem als Vorratsbehälter ausgebildeten Aerosolspender 26 angeschlossen ist. Das Verbindungselement 21 umfasst dabei eine Weiche 22 mit einem Kanal 35, die drei Anschlüsse 23, 24 und 25 aufweist und zwei Schläuche 29 und 30, die an den Anschlüssen 23 und 24 der Weiche 22 angebracht sind. Insbesondere der Schlauch 30, der zwischen der Weiche 22 und dem Nasenraum 3 des Patienten 2 verläuft, soll derart ausgebildet sein, dass eine kapillare Widerstandswirkung aufgrund der Länge und des Durchmessers des Schlauches 30 nicht eintritt. Vielmehr soll die Länge des Schlauches 30 derart ausgebildet sein, dass der Patient 2 das Inhalationsgerät 1 zusammen mit dem Atmungs-Therapiegerät 11 unmittelbar in seinem Gesichtsfeld 7 manuell halten kann. Der Schlauch 30 weist zudem eine hohe Biegesteifigkeit auf, wodurch dieser nicht knickt und folglich das Aerosol 27 ungehindert durchströmen lässt.

Das Atmungs-Therapiegerät 11 ist an dem Anschluss 25 angeschlossen und besteht aus einem Rohr 13, das gekrümmt ausgebildet ist. Die Krümmung des Rohres 13 entspricht im Wesentlichen einem Viertelkreis. Die Verbindung zwischen dem Rohr 13 und dem Anschluss 25 wird durch ein in das Rohr 13 eingestecktes Ansatzstück 14 bewerkstelligt. Das Ansatzstück 14 fixiert gleichzeitig einen Schwingkörper 12, der im Inneren des Rohres 13 freischwingend angeordnet ist. Der Schwingkörper 12 kann als aufblasbarer Schlauch, als Band oder als Kugel, die in einem Ventilsitz angeordnet ist, ausgebildet sein. Im gezeigten Ausführungsbeispiel stellt der Schwingkörper 12 einen Ventilschlauch dar, der beim Ausatmen des Patienten 2 in Schwingung versetzt wird, also gegen die Innenseite des Rohres 13 bewegt wird. Diese Schwingungen erzeugen einen oszillierenden Luftwiderstand, der unmittelbar als oszillierender Überdruck im Nasen-Rachen-Raum 3 bzw. 4 des Patienten 2 wirkt.

Um das Aerosol 27 beim Ausblasen durch das Atmungs-Therapiegerät 11 zu leiten, ist in der Weiche 22 ein Rückschlagventil 32 eingebaut, das beim Einatmen geöffnet und beim Ausatmen geschlossen ist, da die jeweils herrschenden Drücke auf das Rückschlagventil 32 in entsprechender Weise einwirken.

Durch das Ausblasen des Aerosols 27 durch das Atmungs-Therapiegerät 11 entsteht am Ansatzstück 31 aufgrund der Schwingungen des Schwingkörpers 12 ein oszillierend verlaufender Luftwiderstand, der demnach einen oszillierend verlaufenden Überdruck im Nasen-Rachen-Raum 3 bzw. 4 des Patienten 2 erzeugt. Aufgrund des herrschenden oszillierenden Überdruckes im Nasen-Rachen-Raum 3 bzw. 4 sowie in den Nasennebenhöhlen 5 und den jeweiligen Verbindungskanälen 6 im Kopf des Patienten 2 wird das Aerosol 27 in diesen Räumen 3, 4 und 5 und Kanälen 6 optimal verteilt, denn der Überdruck bewirkt, dass beispielsweise ein Verschluss der Nasennebenhöhlen 5 geöffnet wird. Somit kann auch das Aerosol 27 in Räume und Kanäle gelangen, die ursprünglich aufgrund der Erkrankung des Patienten 2 verschlossen gewesen sind.

Dadurch, dass das Aerosol 27 im Wesentlichen im gesamten Nasen-Rachen-Raum 3 bzw. 4 verteilt wird, erhöht sich der Wirkungsgrad des inhalierten Medikamentes, denn dieses gelangt nunmehr auch in Bereiche, die bislang verschlossen gewesen sind. Des Weiteren führt der im Nasen-Rachen-Raum 3 bzw. 4 herrschende Überdruck dazu, dass das Aerosol 27 auch in Schleimhautfalten und Excavationen gelangt. Ohne diesen Überdruck würde nämlich das Aerosol 27 lediglich an der Innenseite der Nasen- und/oder Rachenschleimhäute vorbeiströmen. Das erfindungsgemäße Therapiesystem verbessert somit die Aerosoldespoition in den Schleimhäuten des Nasen-RachenRaumes 3 bzw. 4, wodurch die Therapie der Schleimhäute verbessert ist und wodurch die Resorption von beigemischtem Pharmaka erhöht ist, die über die Nasenschleimhäute aufgenommen werden und anschließend im menschlichen Körper ihre Wirkung entfalten. Demnach können nunmehr Therapien durchgeführt werden, die bislang mit den herkömmlichen Inhalationsgeräten nicht möglich waren.

In Figur 2 wird eine Weiche 22" gezeigt, in die zwei Kanäle 35 und 36 eingeformt sind, der Kanal 35 kommuniziert mit dem Aerosolerzeuger 26 und durch diesen wird demnach das Aerosol 27 in den Nasen- Rachen-Raum 3 eingesogen. Der Kanal 36 mündet in das Atmungs-Therapiegerät 11, so dass die Luft beim Ausatmen durch das Atmungs-Therapiegerät 11 geleitet wird.

Beiden Ausführungsbeispielen der Figuren 1 und 2 ist gemeinsam, dass dem Aerosolspender 26 eine Druck erzeugende Pumpe 34 zugeordnet ist, durch die das Aerosol 27 mittels Überdruck in Richtung des Nasenraumes 3 strömt. Zwischen dem Aerosolspender 26 und dem Verbindungselement 21 ist ein Vernebler 28 eingebaut, durch den das Aerosol 27 in Tropfenform oder als Nebel umgewandelt wird. Zur Ansaugung der Umgebungsluft ist in dem Vernebler 28 ein Einlassventil 33 vorgesehen, durch das die Umgebungsluft eingesaugt wird. Die Durchgangsöffnung des Einlassventils 33 kann von außen variabel eingestellt werden, so dass die Menge der einzusaugenden Umgebungsluft einstellbar ist. Dieses Einlassventil 33 kann auch im Schlauch 29 oder 30 vorgesehen sein.

Das freie Ende des Schlauches 30 mündet in ein Ansatzstück 31, das als Nasenolive ausgebildet ist. Durch dieses Ansatzstück 31 kann der Schlauch 30 in eine Nasenöffnung eingesteckt werden und wird dort luftdicht gehalten. Mit einer Hand fixiert der Patient 2 das Inhalationsgerät 1 in seinem Gesichtsfeld 7 und mit der anderen Hand wird die zweite noch offene Nasenöffnung verschlossen.

Der durch den Aerosolspender 26 erzeugte Überdruck ist dabei derart einzustellen, dass beim Ausatmen durch den Patienten 2 die ausgeatmete Luft durch den Kanal 36 in das Atmungsgerät 11 gelangt. Demnach herrscht beispielsweise im Vernebler 28 oder in der Weiche 22' ein Staudruck vor, der als Widerstand auf die ausgeatmete Luft einwirkt, wodurch diese in den Kanal 36 geleitet wird.

Um die Ausatmungsgeräusche, die insbesondere durch Schwingungen des Schwingkörpers 12 im Rohr 13 entstehen, zu dämpfen, ist am freien Ende des Rohres 13 ein Schalldämpfer 15 eingebaut.

## Patentansprüche

1. Inhalationsgerät (1) zum Einatmen von Luft-Nebel- oder Gas-Gemischen (27), insbesondere von medizinischen Aerosolen, in den Nasen-Rachen-Raum (3 bzw. 4) eines Menschen (2), bestehend aus einem das Aerosol (27) aufnehmenden als Vorratsbehälter ausgebildeten Aerosolspenders (26) und einem an diesen angeschlossenen Verbindungselement (21) durch dessen Kanal (35) das Aerosol (27) dem Nasen- Rachen-Raum (3 bzw. 4) zuführbar ist,
**dadurch gekennzeichnet,**
**dass** an das Verbindungselement (21) ein Atmungs-Therapiegerät (11) angeschlossen ist, dass durch das Atmungs-Therapiegerät (11) beim Ausatmen ein oszillierender Luftwiderstand erzeugbar ist, der im Nasen-Rachen-Raum (3 bzw. 4) einen oszillierenden Überdruck bildet, und dass in dem Kanal (35) des Verbindungselementes (21) zwischen dem Aerosolspender (26) und dem Anschluss für das Atmungs-Therapiegerät (11) ein Rückschlagventil (32) angeordnet ist, das während des Einatmens geöffnet und das während des Ausatmens geschlossen ist.

2. Inhalationsgerät (1) zum Einatmen von Luft-Nebel- oder Gas-Gemischen (27), insbesondere von medizinischen Aerosolen, in den Nasen-Rachen-Raum (3 bzw. 4) eines Menschen (2), bestehend aus einem das Aerosol (27) aufnehmenden als Vorratsbehälter ausgebildeten Aerosolspender (26) und einem an diesen angeschlossenen Verbindungselement (21), durch dessen Kanal (35) das Aerosol (27) dem Nasen-Rachen-Raum (3 bzw. 4) zuführbar ist,
**dadurch gekennzeichnet,**
**dass** an das Verbindungselement (21) ein Atmungs-Therapiegerät (11) angeschlossen ist, dass durch das Atmungs-Therapiegerät (11) beim Ausatmen ein oszillierender Luftwiderstand erzeugbar ist, der im Nasen-Rachen-Raum (3 bzw. 4) einen oszillierenden Überdruck bildet, und dass das Verbindungselement (21) zwei unabhängig voneinander verlaufende Kanäle (33, 36) aufweist, dass der eine Kanal (35) zwischen dem Aerosolspender (26) und dem Nasen-Rachen-Raum (3 bzw. 4) angeordnet ist, in dem ein vorbestimmbarer Überdruck herrscht, und dass der andere Kanal (36) von dem Nasen-Rachen-Raum (3 bzw. 4) in das Therapiegerät (21) einmündet.

3. Inhalationsgerät nach Anspruch 1 oder 2,
**dadurch gekennzeichnet,**
**dass** das Verbindungselement (21) aus einem zweiteiligen Schlauch (29, 30) und einer Weiche (22 bzw. 22') besteht, die drei Anschlüsse (23, 24, 25) aufweist, dass an zwei Anschlüssen (23, 24) der Weiche (22 bzw. 22') jeweils ein Teil des Schlauches (29 bzw. 30) und mit dem dritten Anschluss (25) der Weiche (22 bzw. 22') das Atmungs-Therapiegerät (21) verbunden ist.

4. Inhalationsgerät nach einem oder mehreren
der vorgenannten Ansprüche,
**dadurch gekennzeichnet,**
**dass** der Abstand zwischen der Weiche (22 bzw. 22') und dem Nasen-Rachen-Raum (3 bzw. 4) derart gestaltet ist, dass die Weiche (22 bzw. 22') und/ oder das an dieser angeschlossene Atmungs-Therapiegerät (21) manuell durch den Menschen (2) abstützbar ist.

5. Inhalationsgerät nach einem oder mehreren
der vorgenannten Ansprüche,
**dadurch gekennzeichnet,**
**dass** die Längen und die Durchmesser der beiden an der Weiche (22 bzw. 22') angebrachten Schläuche (29, 30) sowie deren Kanäle (35 bzw. 36) derart ausgebildet sind, dass das Gemisch (27) durch diese während des Ein- und Ausatmens ohne Kapillarenwiderstand bewegbar ist.

6. Inhalationsgerät nach einem oder mehreren der vorgenannten Ansprüche,
**dadurch gekennzeichnet,**
**dass** das Atmungs -Therapiegerät (21) aus einem Rohr (13) gebildet ist, dass in dem Rohr (13) ein Schwingkörper (12) derart angeordnet ist, dass dieser durch das Ausatmen einen oszillierenden Luftwiderstand im Rohr (13) erzeugt.

7. Inhalationsgerät nach Anspruch 6,
**dadurch gekennzeichnet,**
**dass** das Rohr (13) gekrümmt ausgebildet ist und dass die Länge des Rohres (13) in etwa einem Viertelkreis entspricht.

8. Inhalationsgerät nach Anspruch 6 oder 7,
**dadurch gekennzeichnet,**
**dass** der Schwingkörper (12) als Ventilschlauch, als Band oder als Kugel in einem Feder-Ventilsitz ausgebildet ist.

9. lnhalationsgerät nach einem oder mehreren der Ansprüche 6 bis 8,
**dadurch gekennzeichnet,**
**dass** durch den Schwingkörper (12) ein oszillierender Luftwiderstand erzeugbar ist, und dass der Luftwiderstand unmittelbar im Nasen-Rachen-Raum (3 bzw. 4) einen oszillierenden Überdruck erzeugt, durch den die Verteilung des Aerosols (27) gleichmäßig im Nasen-Rachen-Raum (3 bzw. 4) bewerkstelligt ist.

10. Inhalationsgerät nach einem oder mehreren der Ansprüche 6 bis 9,
**dadurch gekennzeichnet,**
**dass** zwischen dem Verbindungselement (21) und dem Rohr (13) des Atmungs - Therapiegerätes (21) ein Ansatzstück (14) in das Rohr (13) eingesteckt ist, durch den der Schwingkörper (14) im Rohr (13) ortsfest gehalten ist.

11. Inhalationsgerät nach einem oder mehreren
der vorgenannten Ansprüche 6 bis 10,
**dadurch gekennzeichnet,**
**dass** an dem freien Ende des Rohres (13) des Atmungs-Therapiegerätes (21) ein Schalldämpfer (15) eingebaut ist, mittels dem die Atemgeräusche dämpfbar sind.

12. Inhalationsgerät nach einem oder mehreren der vorgenannten Ansprüche,
**dadurch gekennzeichnet,**
**dass** dem Aerosolspender (26) eine druckerzeugende Pumpe (34) zugeordnet ist, mittels der das Gemisch (27) in das Verbindungselement (21) einpressbar ist.

13. Inhalationsgerät nach einem oder mehreren der Vorgenannten Ansprüche,
**dadurch gekennzeichnet,**
**dass** in das Verbindungselement (21) ein Vernebler (28) eingesetzt ist, durch den das Gemisch (27) in Tropfenform oder als Nebel umwandelbar ist.

14. Inhalationsgerät nach einem oder mehreren der vorgenannten Ansprüche,
**dadurch gekennzeichnet,**
**dass** in das Verbindungselement (21) ein nach außen mit der Umgebungsluft kommunizierendes Einlassventil (33) eingesetzt ist und dass die von dem Einlassventil (33) frei gegebene Öffnung von außen einstellbar ist.

15. Inhalationsgerät nach Anspruch 14,
**dadurch gekennzeichnet,**
**dass** durch die Einstellung des Einlassventils (33) die in das Verbindungselement (21) eingesaugte Luftmenge vorgebbar ist.

16. Inhalationsgerät nach einem oder mehreren der vorgenannten Ansprüche,
**dadurch gekennzeichnet,**
**dass** an dem Verbindungselement (21) zur luftdichten Einführung des Gemisches (27) in den Nasen-Rachen-Raum (3 bzw. 4) ein Ansatzstück (31) aufsetzbar ist, dessen Außenkontur mit der Innenkontur der Nasenraumöffnung korrespondiert.
Verfahren zur Inhalation von insbesondere medizinischen Gemischen oder Aerosolen (27) in den Nasen-Rachen-Raum (3 bzw. 4) eines Menschen (2), in dem ein an einen als Vorratsbehälter ausgebildeten Aerosolspender (26) angeschlossenes Verbindungselement (21) mit einem Kanal (35) in den Nasen-Rachen-Raum (3 bzw. 4) eingeführt wird,
**gekennzeichnet** t durch die Verfahrensschritte:
- Ausblasen des eingeatmeten Aerosols (27) durch ein Atmungs-Therapiegerät (11), durch das ein oszillierender Luftwiderstand erzeugt wird,
- Erhöhung des Luftdruckes in dem Nasen-Rachen-Raum (3 bzw. 4) durch den von dem Atmungs-Therapiegerät (11) erzeugten oszillierenden Luftwiderstandes und
- Verteilen des Aerosols (27) im Nasen-Rachen-Raum (3 bzw. 4) durch die Druckoszillationen während des Ausatmungsvorganges.
